(19) **Europäisches Patentamt / European Patent Office / Office européen des brevets**

(11) **EP 4 621 061 A1**

(12) **EUROPEAN PATENT APPLICATION**
published in accordance with Art. 153(4) EPC

(43) Date of publication:
**24.09.2025 Bulletin 2025/39**

(21) Application number: **22966155.8**

(22) Date of filing: **24.11.2022**

(51) International Patent Classification (IPC):
*C12P 19/24* $^{(2006.01)}$   *C12P 19/02* $^{(2006.01)}$
*C12N 15/61* $^{(2006.01)}$   *C12N 15/75* $^{(2006.01)}$
*C12N 1/21* $^{(2006.01)}$   *C12R 1/125* $^{(2006.01)}$

(52) Cooperative Patent Classification (CPC):
**C12N 9/90; C12N 15/74; C12N 15/75; C12P 19/02; C12P 19/24;** C12R 2001/125

(86) International application number:
**PCT/CN2022/133876**

(87) International publication number:
**WO 2024/108460 (30.05.2024 Gazette 2024/22)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Anhui Jinhe Industrial Co., Ltd.**
**Chuzhou, Anhui 239200 (CN)**

(72) Inventors:
• **DAI, Yonghui**
**Chuzhou, Anhui 239200 (CN)**
• **QI, Fei**
**Chuzhou, Anhui 239200 (CN)**
• **ZHAO, Yin**
**Chuzhou, Anhui 239200 (CN)**
• **ZHU, Suifan**
**Chuzhou, Anhui 239200 (CN)**

(74) Representative: **Ipside**
**7-9 Allée Haussmann**
**33300 Bordeaux Cedex (FR)**

Remarks:
The complete document including Reference Table(s) and the Sequence Listing(s) can be downloaded from the EPO website

(54) **METHOD FOR PRODUCING PSICOSE BY FERMENTATION AND ISOMERIZATION OF BACILLUS SUBTILIS**

(57) The present invention relates to the technical field of fermentation, and provides a method for producing psicose by fermentation and isomerization of Bacillus subtilis. According to the method of the present invention, fermentation parameters are strictly controlled, and catalytic reaction is performed by using recombinant Bacillus subtilis obtained by fermentation as enzyme liquid, so that the conversion rate of psicose in a system is improved, the problems of low enzyme catalytic reaction efficiency and low yield are solved, and the method is simple to operate and environment-friendly.

EP 4 621 061 A1

FIG.1

## Description

### Technical Field

The present invention relates to the technical field of fermentation, and particularly relates to a method for producing psicose by fermentation and isomerization of Bacillus subtilis.

### Background of the Invention

D-psicose (D-allulose or D-psicose, hereinafter referred to as psicose) is a rare sugar which is present in small amounts in wheat, murraya, and processed sugar cane and sugar beet syrups; in addition, psicose is also present in a small amount in a mixed syrup of fructose and glucose. In the heat treatment of high-sugar foods, psicose may be produced by conversion from fructose by a non-enzymatic reaction, and thus it is also present in a trace amount in processed foods such as sauces, juices, sugar cane and beet syrup.

Psicose has many specific physiological properties, compared with glucose, psicose has a lower absorption rate in human body, and competes with glucose and fructose at cell membrane surface transporters, thereby reducing fructose and glucose absorption in the daily diet. The special physiological properties of psicose make it of great significance to human health, such as: enhancing insulin resistance, inhibiting postprandial blood glucose increase, reducing fat accumulation in abdomen, and preventing diabetes. In addition, psicose has antiinflammatory, nerve protecting, active oxygen free radical scavenging, and arteriosclerosis treating effects. In 2014, psicose was recognized by the U.S. food and drug administration as a safe food (GRAS). The special physiological properties of psicose make it important for use in the fields of medicine and health food.

At present, the production method of psicose comprises a biological enzyme method and a fermentation method, but the fermentation method has low enzyme activity, so that the yield is low, the operation is complex, the production cost is high, and the requirement of industrial production cannot be met.

### Summary of the Invention

The invention aims to provide a method for producing psicose by fermentation and isomerization of Bacillus subtilis, which can greatly improve the activity of D-psicose-3-epimerase produced by the Bacillus subtilis so as to improve the conversion rate of the psicose, simplifies the operation and is suitable for popularization and application in industrial large-scale production of psicose.

The invention provides a method for producing psicose by fermentation and isomerization of Bacillus subtilis, which comprises the following steps:

Inoculating recombinant Bacillus subtilis 1A751-DPE seed liquid for expressing D-psicose 3-epimerase into a fermentation culture medium for fermentation culture to obtain fermentation liquid; during the fermentation culture period, the aeration ratio is 0.1-2 V/V.min; the rotation speed of the fermentation culture is 200-1000 rpm;

Separating thalli from the fermentation liquid;

Carrying out catalytic reaction on a substrate fructose by the thalli to obtain the psicose.

Preferably, the fermentation medium comprises the following components: 10-40 g/L of bean cake powder, 20-50 g/L of bran, 30-60 g/L of corn flour, 1-10 g/L of dipotassium hydrogen phosphate, 1-10 g/L of magnesium sulfate and 0.1-5 g/L of manganese sulfate, wherein the pH value of the fermentation medium is 6-8.

Preferably, the fermentation medium comprises the following components: 20-35 g/L of bean cake powder, 35-45 g/L of bran, 45-55 g/L of corn flour, 4-8 g/L of dipotassium hydrogen phosphate, 5-8 g/L of magnesium sulfate and 1-3 g/L of manganese sulfate, wherein the pH value of the fermentation medium is 6.5-7.5.

Preferably, the rotation speed of the fermentation culture is 200-1000 rpm, and the aeration ratio is 0.1-2V/V.min;

The temperature of the fermentation culture is 30-37 °C, and the time of the fermentation culture is 48-72 h;

During fermentation, the pH value of fermentation system is controlled to be 6-8;

The inoculation amount of the recombinant Bacillus subtilis 1A751-DPE seed liquid for expressing the D-psicose 3-epimerase is 1%-10%.

Preferably, the system of the catalytic reaction is 1-5 ml of suspension of the thalli, 50-150 g of fructose and 50-200 ml of water;

The thalli concentration of the thalli suspension is $(30-48) \times 10^9$ CFU/ml.

Preferably, the temperature of the catalytic reaction is 50-60 °C;

The time of the catalytic reaction is 2-4 h.

Preferably, the culture medium of the recombinant Bacillus subtilis 1A751-DPE seed liquid for expressing the D-psicose 3-epimerase is an LB culture medium;

The culture temperature of the recombinant Bacillus subtilis 1A751-DPE seed liquid for expressing the D-psicose 3-epimerase is 30-37 °C;

The culture time of the recombinant Bacillus subtilis 1A751-DPE seed liquid for expressing the D-psicose 3-epimerase is 20-24 h;

The rotation speed of the recombinant Bacillus subtilis 1A751-DPE seed liquid for expressing the D-psicose 3-epimerase is 200-400 rpm;

When the recombinant Bacillus subtilis 1A751-DPE seed liquid for expressing the D-psicose 3-epimerase is cultured, the aeration ratio is 0.1-1 V/V.min;

The inoculation amount of the recombinant Bacillus subtilis 1A751-DPE seed liquid for expressing the D-psicose 3-epimerase is 1%-10%.

Preferably, the method for separating the thalli is ceramic membrane ultrafiltration;
The pore diameter of the ceramic membrane for ultrafiltration is 0.22 nm or less.

The invention provides a method for producing psicose by fermentation and isomerization of Bacillus subtilis, wherein recombinant Bacillus subtilis 1A751-DPE obtained by fermentation is used as an enzyme solution to perform catalytic reaction on fructose, and the aeration ratio and the rotation speed during fermentation culture are strictly controlled, so that the enzyme activity of D-psicose 3-epimerase of the recombinant Bacillus subtilis 1A751-DPE is favorably improved, and the conversion rate of psicose is improved. The results of the examples show that the highest conversion rate of the psicose in the system can reach 31.4%; the method effectively solves the problems of low efficiency and low yield of the enzyme catalysis reaction, is simple to operate and environment-friendly, and has important market value and application value. Meanwhile, the host bacteria in the invention are Bacillus subtilis, so that the food safety performance is high.

The method provided by the invention specifically limits the fermentation conditions and the culture medium, and has the following advantages:

According to the method for preparing the psicose, the fermentation is carried out by one-time feeding, so that the catalytic reaction efficiency is improved, the operation steps are reduced, the production cost is reduced, the industrial production is easy, and the problems of low enzyme activity, complex operation and high production cost of the existing fermentation method are solved; in addition, the bean cake powder, the bran, the corn flour and the like used in the fermentation medium are cheap and widely available raw materials, so that the production cost is greatly reduced. The fermentation method provided by the invention catalyzes the production of psicose, the enzyme activity level high, the conversion rate is over 29.3% and is far higher than the existing reported yield, the production cost is reduced, and the industrial production is facilitated.

**Brief Description of the Drawings**

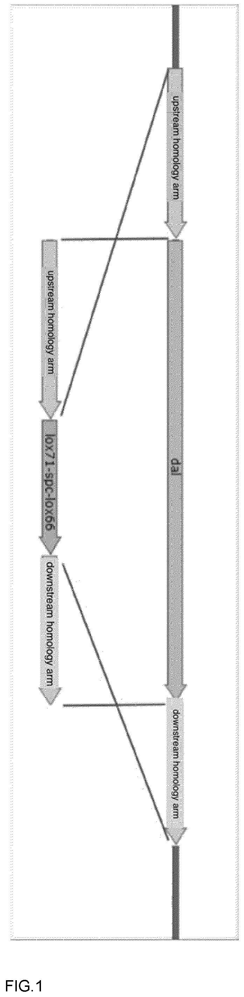

FIG. 1 is a schematic diagram showing chromosomal dal gene knock-out of Bacillus subtilis;

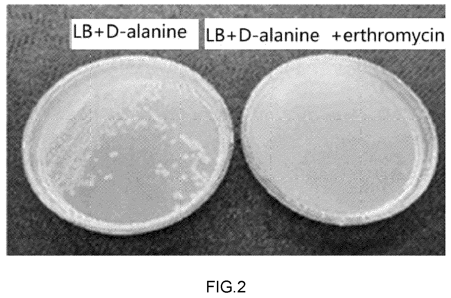

FIG. 2 shows the result of verifying the loss of the thermo-sensitive plasmid pTSC;

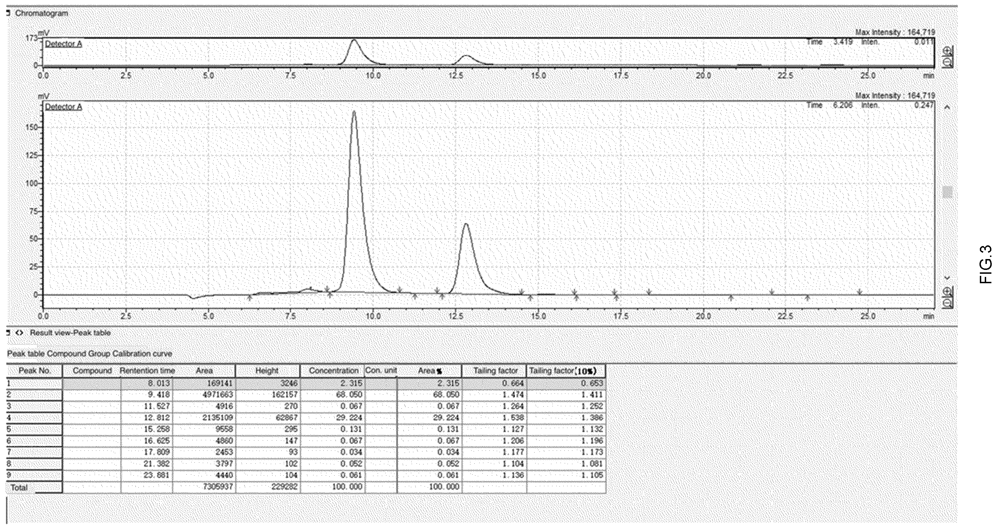

FIG. 3 shows the results of detection of psicose in example 2;

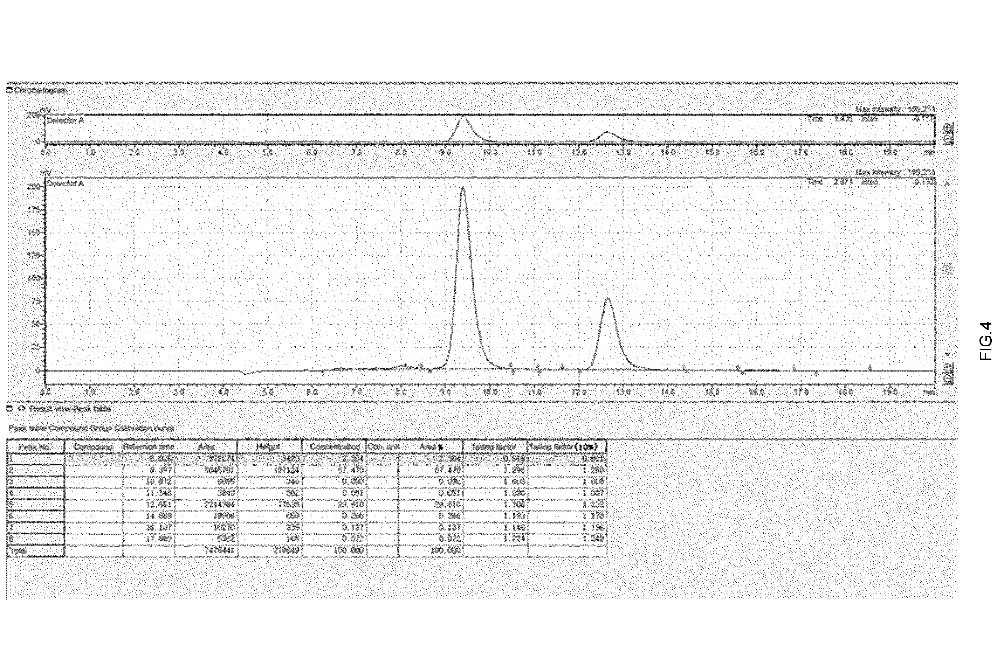

FIG. 4 shows the results of detection of psicose in example 3;

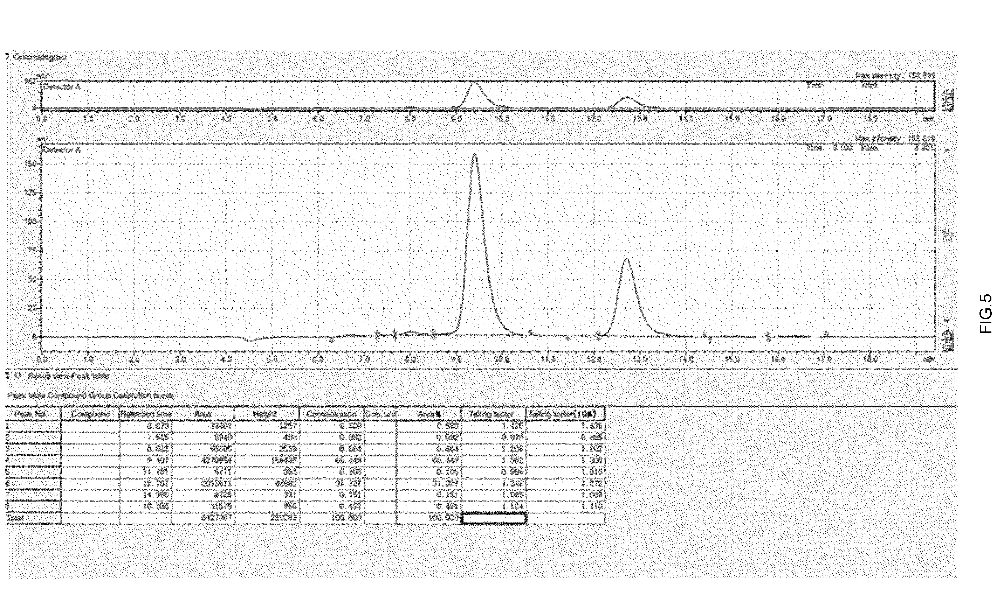

FIG. 5 is the result of detection of psicose in example 4.

**Detailed Description of Embodiments**

The invention provides a method for producing psicose by fermentation and isomerization of Bacillus subtilis, which comprises the following steps:

Inoculating recombinant Bacillus subtilis 1A751-DPE seed liquid for expressing D-psicose 3-epimerase into a fermentation culture medium for fermentation culture to obtain fermentation liquid; during the fermentation culture period, the aeration ratio is 0.1-2 V/V.min; the rotation speed of the fermentation culture is 200-1000 rpm;
Separating thalli from the fermentation liquid;
Carrying out catalytic reaction on a substrate fructose by the thalli to obtain the psicose.

**EP 4 621 061 A1**

According to the invention, recombinant Bacillus subtilis 1A751-DPE seed liquid for expressing D-psicose 3-epimerase is inoculated into a fermentation medium for culture to obtain fermentation liquid.

In the present invention, recombinant Bacillus subtilis 1A751-DPE for expressing D-psicose 3-epimerase is constructed by preferably knocking out the dal gene (expressing D-alanine racemase) of Bacillus subtilis 1A751 by homologous recombination technique, and simultaneously transferring the recombinant plasmid pUB-P43dpe-dal expressing D-psicose 3-epimerase (DPE), and the recombinant Bacillus subtilis 1A751-DPE was developed to efficiently express D-psicose 3-epimerase.

In the present invention, the culture medium for the recombinant Bacillus subtilis 1A751-DPE seed liquor expressing the D-psicose 3-epimerase is preferably LB medium. The culture temperature of the recombinant Bacillus subtilis 1A751-DPE seed liquor expressing the D-psicose 3-epimerase is preferably 30-37 °C, more preferably 32-35 °C, and most preferably 33 °C. The culture time of the recombinant Bacillus subtilis 1A751-DPE seed liquor expressing the D-psicose 3-epimerase is preferably 20-24 h, more preferably 21-23 h, and most preferably 22 h. The culture rotation speed of the recombinant Bacillus subtilis 1A751-DPE seed liquor expressing the D-psicose 3-epimerase is preferably 200-400 rpm, more preferably 250-350 rpm, and most preferably 300 rpm. When the recombinant Bacillus subtilis 1A751-DPE seed liquor expressing the D-psicose 3-epimerase is cultured, the aeration ratio is preferably 0.1-1 V/V.min, more preferably 0.2-0.8 V/V.min, most preferably 0.4-0.7 V/V.min, and most preferably 0.5 V/V.min. The aeration ratio is the ratio of the volume of air passing through a unit volume of culture solution per minute. The inoculation amount of the recombinant Bacillus subtilis 1A751-DPE seed liquor expressing the D-psicose 3-epimerase is preferably 1%-10%, more preferably 2%-8%, further preferably 4%-7%, and most preferably 5%. The preparation of the recombinant Bacillus subtilis 1A751-DPE seed liquor expressing the D-psicose 3-epimerase is preferably carried out in a seed tank. The volume of the seed tank is preferably 30-70 L, and more preferably 50 L.

In the present invention, the fermentation medium preferably comprises the following components: 10-40 g/L of bean cake powder, 20-50 g/L of bran, 30-60 g/L of corn flour, 1-10 g/L of dipotassium hydrogen phosphate, 1-10 g/L of magnesium sulfate and 0.1-5 g/L of manganese sulfate, wherein the pH value of the fermentation medium is 6-8, and the fermentation medium preferably comprises the following components: 20-35 g/L of bean cake powder, 35-45 g/L of bran, 45-55 g/L of corn flour, 4-8 g/L of dipotassium hydrogen phosphate, 5-8 g/L of magnesium sulfate and 1-3 g/L of manganese sulfate, wherein the pH value of the fermentation medium is 6.5-7.5.

In the present invention, the rotation speed of the fermentation culture is preferably 200-1000 rpm, more preferably 400-800 rpm, and further preferably 500 rpm. The aeration ratio is preferably 0.1-2 V/V.min, more preferably 0.2-1.8 V/V.min, still more preferably 0.5-1.5 V/V.min, and most preferably 1.0 V/V.min. The temperature of the fermentation culture is preferably 30-37 °C, more preferably 32-35 °C, and most preferably 34 °C. The time for fermentation culture is preferably 48-72 h, more preferably 52-68 h, further preferably 54-65 h, and most preferably 60 h. During the fermentation, the pH value of the fermentation system is preferably controlled to be 6-8, more preferably 6.5-7.5, and most preferably 7.0. The inoculation amount of the recombinant Bacillus subtilis 1A751-DPE seed liquor expressing the D-psicose 3-epimerase is preferably 1%-10%, more preferably 2%-8%, even more preferably 4%-7%, and most preferably 5%. The cultivation is preferably carried out in a fermenter. The volume of the fermenter is preferably 250-750 L, more preferably 350-700 L, and further preferably 500 L.

After the fermentation liquid is obtained, the invention separates the thalli from the fermentation liquid.

In the present invention, the method for separating the thalli is preferably ceramic membrane ultrafiltration. The pore diameter of the ceramic membrane for the ceramic membrane ultrafiltration is preferably 0.22 nm or less, more preferably 0.2 nm.

After obtaining the thalli, the thalli are subjected to catalytic reaction on a substrate fructose to obtain the psicose.

In the present invention, the system of the catalytic reaction is preferably 1-5 ml of suspension of the thalli, 50-150 g of fructose and 50-200 ml of water; the water is preferably deionized water. The thalli concentration of the thalli suspension is $(30-48)\times10^9$ CFU/ml, and more preferably $42\times10^9$ CFU/ml. The temperature of the catalytic reaction is preferably 50-60 °C, more preferably 52-58 °C, and most preferably 55 °C. The time of the catalytic reaction is preferably 2-4 h, and more preferably 3 h.

In the present invention, the fermentation liquid is centrifugally filtered to remove impurities, transferred into a liquid phase small bottle and analyzed by high performance liquid chromatography, and the analysis conditions of the high performance liquid chromatography are as follows: chromatographic column: Carbpmix Ca-NP; mobile phase: ultrapure water (pre-sonication to exclude air); flow rate: 0.6 mL/min; column temperature: 85 °C; detector: differential refractometer: temperature of the detector: 30 °C; sample injection amount: 10 μL. Analyzing the proportion of the psicose peak area according to a peak diagram area normalization method of liquid chromatogram to obtain the conversion rate of the psicose.

The method for producing psicose by fermentation using Bacillus subtilis provided by the present invention is described in detail below with reference to examples, but they should not be construed as limiting the scope of the present invention.

Example 1

Construction method of recombinant Bacillus subtilis 1A751-DPE

1. The primers required in the construction process are shown in Table 1, and the primers are synthesized by Sangon Biotech (Shanghai) Co., Ltd.

Table 1. Primers required for the construction procedure

| Primers | Sequence (5'->3') |
|---|---|
| P1 | TACAAAGCAAAAGCGAAAATGACCATC (SEQ ID NO:1) |
| P2 | ATGCTATACGAACGGTACTTAGAAGAGAGAAGGTAA (SEQ ID NO:2) |
| P3 | TTACCTTCCTTTTAAGTACCGTTCGTATAGCAT (SEQ ID NO:3) |
| P4 | CAAGCAAAGCTGTTTTATCTACCGTTCGTATAATGT (SEQ DI NO:4) |
| P5 | ACATTATACGAACGGTAGATAAAACAGCTTTGCTTG (SEQ ID NO:5) |
| P6 | CAGCTGATAGGATTCTTGCTCGCTTA (SEQ ID NO: 6) |
| P7 | CGTCTGTACGTTCCTTAAGGAATTCTTAGGAGTGTTTATGACATTCTAAT (SEQ ID NO:7) |
| P8 | TAGAATGCAAAAAGTGAAATCATAATGATAGGTGGTATGTTTTCGCTTGA (SEQ ID NO:8) |
| P9 | ATTAGAATGTCATAAACACTCCTAAGAATTCCTTAAGGAACGTACAGACG (SEQ ID NO:9) |
| P10 | TCAAGCGAAAACATACCACCTATCATTATGATTTCACTTTTTGCATT (SEQ ID NO:10) |
| P11 | ATTAACTTACCTAAATGGAGAATTCAATCTATTATTAATCTGTTCAGCAA (SEQ ID NO:11) |
| P12 | CACCATTTCCCTTTAGAAAGATCTTCCCTTTTCAGATAATTTTAGATTT (SEQ ID NO:12) |
| P13 | AAATCTAAAATTATCTGAAAAGGGAAGATCTTTCTAAAGAGGAAATGGTG (SEQ ID NO: 13) |
| P14 | TTGCTGAACAGATTAATAATAGATTGAATTCTCCATTTAGGTAAGTTAAT (SEQ ID NO: 14) |

2. Construction of recombinant Bacillus subtilis 1A751(dal-)

2.1 Extraction of Bacillus subtilis 1A751 genome

**The** extraction of the genome of B. subtilis 1A751 refers to an operation manual of an Ezup column type bacterial genome DNA extraction kit (Sangon Biotech (Shanghai) Co., Ltd.), and the specific steps are as follows:

(1) A single B.subtilis 1A751 colony was picked from a fresh LB plate, inoculated into 4 mL of LB liquid medium, and cultured overnight on a shaker at 37 °C at 200 r/min.

(2) 1 mL of B.subtilis 1A751 bacterial liquid was sucked, and the bacterial was separated by centrifugation at 8000 r/min for 1 min. Added 180 $\mu$L of 20 mg/mL lysozyme solution prepared in advance to resuspend the thalli, carried out water bath at 37 °C for 60 min, then added 400 $\mu$L of Buffer Digestion, shaked and mixed uniformly. Finally, the mixture was kept in a water bath at 65 °C for 60min (evenly mixed by reversing every 10 min in the process) until the mixture became clear and transparent (cells were completely lysed).

(3) Added 20 $\mu$L of 10mg/mL RNaseA, and standed at room temperature for 2-5 min.

(4) Added 200 $\mu$L Buffer PB and mixed well by inversion, -left at-20 °C for 5 min.

(5) Centrifugation was carried out at 10000 r/min for 5 min, and the supernatant was transferred to a new 1.5 mL centrifuge tube.

(6) Added isopropanol with the same volume, and reversed for 5-8 times to fully and uniformly mix. Standed at room temperature for 2-3min, centrifuged at 10000 r/min for 5min, and discarded the supernatant.

(7) Suspended and precipitated with 1mL of 75% ethanol, rinsed by inversion for 1-3 min, centrifuged at 10000r/min for 2min, and discarded the supernatant.

(8) Repeated step (7) once.

(9) Opened the cover and standed for 5-10 min to completely volatilize the residual ethanol.

(10) Dissolved with 50 $\mu$L **TE** Buffer (10mmol/L Tris/HCl, 1mmol/L EDTA, pH 8.0). **The** resulting DNA solution can be subjected to the next experiment or stored at-20 °C.

2.2 Amplification of homology arms

Fragments with the length of 800-900 bp are respectively selected from two sides of a D-alanine racemase gene (GenBank No. CAB12271.1) on a chromosome of the Bacillus subtilis 1A751 to be used as homologous arms, and amplification primers are designed. **The** upstream homology arm (SEQ ID NO:15, tacaaagcaaaagcgaaaatgaccatcgag acagggagtgagcctcaagtatacaatgtggaaatctggcacaaaaagcc gtctctgtacagggtttatttagaaaatccgaaaaaggaccaaaaccaagtcattt tacgaaatgaaaacggagtattcgttctca cgccgtctttaaataagagcttcagatttcaaagcgattggccgaacaacagcagccaggtttatctgtttgaatcgctc gtaaagg atgttcaaaacgattcggatgcagttttcacagcgaaagaaaagaaatacgtatttgaaacaaaaacaaactatcagcataaca aaatgctgcccac gcaagaaatcacatttaacaaaaaagatatgagcccgtcatctgtcaaagtgatggatactgaccggaaa gtgatggtcaaagtagaattcagcagctttgaattta ataaacaatttgataaagaatcatttgatgaaaagaaaaatatgaccctt ctcaaatggatgtcgccacaagcgcaaagccttccgatacatttgcggtcaaaacgc cgctggaactgccgcttggcgtcaagc tgcttgaagaaaaagatatatctactgaagacgggaagcgcatcatcatgacgtacggaggagaaaaatcatttacgtt aattca ggaaaaagcccagattgccaaggcttcctcctccgttacgctgaacggcgaaccggtaaatctcggctacaccatcggcgccct gtcggatgcatcattat catggacatatgacggcgtagattaccttctctcttctaa) was amplified by the primer pair P1 and P2, and the downstream homology arm (SEQ ID NO: 16, ataaaacagctttgcttgaagagtgaataatggtatcattatcactggatggataaaagagtcgtatccgtaagtttggtggaggtgt at gttttttgtctgaatccagcgcaagaaccgaaatgaaaatcagcttgcccgaaaacctagtagctgaattggatggtgtagcga tgcgggagaaacgaagcagaa acgaactgatatcacaagcagtgagagcgtatgtcagcgaacgaacaactcgtcacaac cgtgatttgatgagacgcggctatatggaaatggcgaaaatcaac ctgaatatttcttctgaggctcactttgcggagtgcgaggct gaaacgacagttgagcgcttagtcagcggaggataatcatttgattgtgaaacgcggcgatgtttatttt gctgatttatctcctgttgtt ggctcagagcaaggcggggtgcgcccggttttagtgatccaaaatgacatcggaaatcgcttcagcccaactgctattgttgcag cca- taacag cacaaatacagaaagcgaaattaccaacccacgtcgaaatcgatgcaaaacgctacggtttgaaagagattc cgttattttgctggagcaaattcggac gattgacaagcaaaggttaacggataagattactcatctggatgatgaaatgatggata aggttgatgaagccttacaaatcagtttggcactcattgattttttagacat atttgcaggttgctcaaatagagcaacttttttttgtttttcaa aaaacataaacgatataatagtgaaataacgaaaaaatatgttgttttttattgggaggtaagcgaatttg atgtcgaaccagactg tataccagttcattgccgaaaatcaaaatgaactgctccaactgtggactgacacactaaaagaattaagcgagcaagaatcct at- cagctg) was amplified by the primer pair P5 and P6, using the genome of Bacillus subtilis 1A751 as a template. PCR amplification products are respectively recovered and purified and the DNA concentration was measured.

Amplification of the lox71-spc-lox66 resistance gene expression cassette

An upstream primer P3 and a downstream primer P4 are respectively designed according to the sequence of the lox71-spc-lox66 resistance gene expression cassette, and the primers P3 and P4 are respectively reverse complementary with the sequences of the primers P2 and P5. The plasmid p7S6 is used as a template, primers P3 and P4 are used for amplification to obtain a lox71-spc-lox66 resistance gene expression cassette, and PCR amplification product gel is recovered and purified, and the DNA concentration is measured.

1.4 Fusion of lox71-spc-lox66 resistance gene expression cassette and upstream, downstream homology arms

The fusion of the three DNA fragments adopts a two-step PCR method (Shevchuk N A et al, 2004), and the specific operation steps are as follows:

The first PCR reaction system is shown in Table 2.

Table 2. PCR reaction System

| | |
|---|---|
| Phusion HF Buffer (5×) | 5 μL |
| dNTP Mixture (2.5 mmol/L each) | 2 μL |
| Upstream homologous arm fragments (40 ng/μL) | 2.5 μL |
| *lox71-spc-lox66* fragment (40 ng/μL) | 0.5 μL |
| Downstream homologous arm fragments (40 ng/μL) | 2.5 μL |
| Phusion High-Fidelity DNA Polymerase | 0.5μL |
| ddH$_2$O | Supplement the system with 25 μL |

PCR reaction conditions:

Performing pre-denaturation at 98 °C for 2 min; melting at 98 °C for 10 s, annealing at 56 °C for l5 s, and extensing at 72 °C for 2min for 10 cycles; extensing at 72 °C for 5 min.

The second PCR reaction system is shown in Table 3.

Table 3. PCR reaction System

| | |
|---|---|
| Phusion HF Buffer (5×) | 10 μL |
| dNTP Mixture (2.5 mmol/L each) | 4 μL |

(continued)

| | |
|---|---|
| Primer P1 | 1.25 μL |
| Primer P6 | 1.25 μL |
| PCR product in the first step | 2 μL |
| Phusion High-Fidelity DNA Polymerase | 0.5 μL |
| ddH$_2$O | Supplement the system with 50 μL |

PCR reaction conditions:

Performing pre-denaturation at 98 °C for 2 min; melting at 98 °C for 10 s, annealing at 60 °C for I5 s, and extensing at 72 °C for 2min for 30 cycles; extensing at 72 °C for 5 min.

2.5 Preparation and transformation of competence of Bacillus subtilis

Bacillus subtilis competent cells were prepared by a two-step (GM I, GM II) method (Julkowska D et al, 2005). The specific operation steps are as follows:

(1) The B.subtilis strain stored in an ultralow temperature refrigerator was streaked on a spore slant, and placed in an incubator at 37 °C for static culture for 24-48 h.
(2) A loopful of bacterial liquid was picked and inoculated into 5mL of GM I medium and cultured overnight in a shaker at 37 °C at 200 r/min.
(3) The next day, 2 mL of GM I culture was transferred to 18mL of GM I medium and cultured at 37 °C at 200 r/min for 3.5 h.
(4) 10 mL of the culture was transferred to 90 mL of GM II medium, cultured at 37 °C for 1.5 h at 100 r/min, and centrifuged at 6000 r/min for 10 min to separate the thalli.
(5) The thalli suspension was gently suspended in 10 mL of supernatant, and the mixed solution was dispensed into sterile 1.5 mL centrifuge tubes at 500 μL/tube and used directly for transformation, or 50% glycerol was added to a final concentration of 10% and stored immediately in a -80 °C ultra-low temperature freezer.
(6) During transformation, putting the centrifugal tube into a water bath at 45 °C for melting, a proper amount of recombinant plasmid or DNA fragment was added, and gently mixed.
(7) Resuscitated at 37 °C at 100 r/min for 30 min, spreaded plate containing corresponding antibiotics, and cultured in 37 °C incubator by inverting overnight.

2.6 Screening of recombinant Bacillus subtilis 1A751(dal-, Spcr)

Bacillus subtilis 1A751 competence was prepared according to the method of step 2.5, the fusion fragment obtained in step 2.4 is transformed into the competence, and finally spreaded on plates to which both D-alanine and spectinomycin were added, and transformants were picked the next day and spotted on LB plates and plates to which both D-alanine and spectinomycin were added for further selection.

2.7 Knock-out of antibiotic resistance Gene

(1) Using recombinant Bacillus subtilis 1A751(dal-, Spcr) as a host bacterium, competence was prepared in reference to step 2.5, and D-alanine solution was additionally added to the competent medium GM I and GM II to give a final concentration of 200 μg/mL.
(2) The plasmid pTSC was transformed into the above-mentioned Bacillus subtilis competence, spreaded on LB plates containing erythromycin and D-alanine, and subjected to inverted culture at 37 °C for 12 h in an incubator.
(3) Transformants were selected from the plate, and inoculated into an LB liquid culture medium containing erythromycin and D-alanine, performed shake culture at 37 °C by using a shaking table, and added IPTG for induction for 6 h when OD$_{600}$ is 0.6-0.8. A loopful of bacterial liquid was picked up using an inoculating ring, and marked on an LB plate which is free of the antibiotics and added with D-alanine.
(4) Single colonies were selected from the overnight-cultured plate, and dropped on LB plate added with D-alanine and LB plate simultaneously added with spectinomycin and D-alanine, and carried out overnight culture to screen out colonies which do not grow on the spectinomycin plate and grow on the corresponding LB plate containing D-alanine.
(5) The obtained recombinant bacterium was cultured for 2 days at 51 °C, so that the temperature-sensitive plasmid pTSC was lost. The resulting D-alanine auxotrophic mutant strain was designated B.subtilis 1A751 (dal-).

3. Introduction of foreign plasmid

3.1 Construction of recombinant plasmid

The plasmid pUB110 contains the Hpa II promoter, a Bacillus subtilis endogenous promoter P43 was added based on the Hpa II promoter. Recombinant plasmid construction procedures refer to the "Simple cloning" method (You et al, 2012). The specific operation steps are as follows:

(1) Amplified the target gene of P43-dpe. The plasmid T-P43-dpe was used as a template, primers P7 and P8 were used for amplification, and PCR product gel was recovered and purified and its DNA concentration was measured.
(2) The vector skeleton was amplified with primers P9 and P10 using plasmid pUB110 as template, PCR product gel was recovered and purified and its DNA concentration was measured.
(3) The reagents in table 4 were added to a microcentrifuge tube, and PCR reaction conditions are as follows:

Performing pre-denaturation at 98 °C for 2 min; melting at 98 °C for 10 s, annealing at 60 °C for I5 s, and extensing at 72 °C for 2 min for 30 cycles; extensing at 72 °C for 5 min.

Table 4. PCR reaction System

| | |
|---|---|
| Phusion HF Buffer (5×) | 10 μL |
| dNTP Mixture (2.5 mmol/L each | 5 μL |
| P43-dpe gel recycled fragments | 2 μL |
| Vector skeleton gel recycled fragment (100 ng/μL) | 2 μL |
| Phusion High-Fidelity DNA Polymerase | 0.5 μL |
| H$_2$O | Supplement the system with 50 μL |

(4) The PCR product (multimeric fragment) was transformed into Bacillus subtilis 1A751 competence, and plated on LB plate containing kanamycin for selection.
(5) Transformants were picked and inoculated into LB liquid medium, and plasmids were extracted the next day. Plasmids were extracted from Bacillus subtilis, referring to a small extraction kit (Sangon Biotech (Shanghai) Co., Ltd.) operation manual of plasmid DNA, suspending thalli by using Buffer P1 in the first step, adding 20 mg/mL lysozyme, keeping the temperature at 37 °C for 30 min, then adding Buffer P2, and the subsequent steps are the same as the operation manual. The recombinant plasmid obtained by extraction was designated pUB-P43dpe.

3.2 Replacement of antibiotic resistance gene by D-alanine racemase gene

(1) Recombinant plasmid pUB-P43dpe was used as a template, primers P11 and P12 were used to amplify the vector skeleton sequence of the antibiotic resistance gene removed part, and the obtained PCR product gel was recovered and purified.
(2) Primers P13 and P14 were used to amplify the D-alanine racemase gene by using the B.subtilis 1A751 genome as a template, and PCR product gel was recovered and purified.
(3) The D-alanine racemase gene and the corresponding vector skeleton fragment are subjected to PCR, and a recombinant plasmid based on the dal gene screening marker is constructed by using "Simple cloning" method. Fragments of the multimer were transformed into D-alanine deficient Bacillus subtilis 1A751(dal-) competence, transformed into 1A751(dal-) strain competence due to the fact that the plasmid carried the dal gene, and recombinant plasmid selection was achieved by selection on LB plates without the addition of D-alanine.
(4) The resulting recombinant plasmid was designated pUB-P43dpe-dal.

3.3 Construction of recombinant Bacillus subtilis

In the experiment, recombinant plasmids were directly constructed in Bacillus subtilis, so that the positive clone transformant containing the recombinant plasmids, which is obtained by screening in step 3.2, is the recombinant Bacillus subtilis 1A751/pUB-P43dpe-dal, and the transformant is designated 1A751-DPE.

Example 2

A method for producing psicose by fermentation and isomerization of Bacillus subtilis comprises the following specific implementation steps:

(1) Seed preparation: inoculating the Bacillus subtilis implanted with the Closridium gene prepared in example 1 into an LB medium, and culturing for 20 h at 37 °C and 100 rpm;
(2) Cultivation in a 50 L seed tank: inoculating the Bacillus subtilis obtained in step (1) into a seed tank filled with 35 L of LB culture medium according to the inoculation amount of 5% of the volume ratio for culturing, controlling the rotation speed of the seed tank to be 300rpm, controlling the aeration ratio to be 0.5 V/V.min, and culturing at 35 °C for 20 h to obtain seed liquid;
(3) Production in a fermentation tank: inoculating the seed liquid into a fermentation tank filled with a fermentation culture medium for culture according to the inoculation amount of 5% volume ratio, wherein the preparation method of the fermentation culture medium comprises the steps of mixing 10 g of bean cake powder, 20 g of bran, 30 g of corn flour, 1 g of dipotassium phosphate, 1 g of magnesium sulfate and 0.1 g of manganese sulfate, adjusting the pH to 7 by using a sodium hydroxide solution, and fixing the volume to 1000 ml by using deionized water; controlling the rotation speed of the fermentation tank to be 500 rpm, controlling the aeration ratio to be 1 V/V.min, controlling the pH to be 7.5 at the temperature of 30 °C, culturing for 60 h, and obtaining fermentation liquid after fermentation;
(4) Carrying out ultrafiltration on the fermentation liquid obtained in step (3) by adopting a ceramic membrane with a pore diameter of 0.22 nm to obtain Bacillus subtilis thalli and obtain enzyme liquid;
(5) An isomeric system: 5 ml of the enzyme solution was mixed with 114 g of fructose and 109 ml of deionized water, followed by reaction at 55 °C for 4 h. And after the reaction is finished, heating the reaction solution to inactivate enzyme, centrifugally filtering the sample, performing liquid chromatography analysis treatment, and analyzing the area ratio of the psicose peak according to a peak map area normalization method of the liquid chromatography to obtain the conversion rate of the psicose.

**The** technical scheme is adopted to produce psicose by fermentation, and the conversion rate of psicose is 29.3%.

Example 3

A method for producing psicose by fermentation and isomerization of Bacillus subtilis comprises the following specific implementation steps:

(1) Seed preparation: inoculating the Bacillus subtilis implanted with the Closridium gene prepared in example 1 into an LB medium, and culturing for 22 h at the temperature of 35 °C and rotation speed of 200 rpm;
(2) Cultivation in a 50 L seed tank: inoculating the Bacillus subtilis obtained in step (1) into a seed tank filled with 35 L of LB culture medium according to the inoculation amount of 8% of the volume ratio for culture, controlling the rotation speed of the seed tank to be 400 rpm, controlling the aeration ratio to be 1 V/V.min, and culturing at the temperature of 30 °C for 24 h to obtain seed liquid;
(3) Production in a 5000 L fermentation tank: inoculating seed liquid into a fermentation tank filled with 3500 L of fermentation medium for culture according to the inoculation amount of 8% volume ratio, wherein the preparation method of the fermentation medium comprises the steps of mixing 20 g of bean cake powder, 40 g of bran, 55 g of corn flour, 4 g of dipotassium hydrogen phosphate, 6 g of magnesium sulfate and 3.5 g of manganese sulfate, adjusting the pH to 7.2 by using a sodium hydroxide solution, and fixing the volume to 1000 ml by using deionized water; controlling the rotation speed of the fermentation tank to be 500 rpm, controlling the aeration ratio to be 1.5 V/V.min, controlling the pH to be 8 at the temperature of 30 °C, culturing for 72 h, and obtaining fermentation liquid after the fermentation is finished;
(4) Carrying out ultrafiltration on the fermentation liquid obtained in step (3) by adopting a ceramic membrane with a pore diameter of 0.22 nm to obtain Bacillus subtilis thalli and obtain enzyme liquid;
(5) An isomeric system: 5ml of the enzyme solution was mixed with 114 g of fructose and 109 ml of deionized water, followed by reaction at 60 °C for 3 h, and the psicose conversion was measured and calculated according to the method of example 2. **The** result shows that the conversion rate of the psicose produced by the technical scheme is 29.8%.

Example 4

A method for producing psicose by fermentation and isomerization of Bacillus subtilis comprises the following specific implementation steps:

(1) Seed preparation: inoculating the Bacillus subtilis implanted with the Closridium gene prepared in example 1 into an LB medium, and culturing for 24 h at the temperature of the LB medium temperature of 37 °C and rotation speed of 250 rpm;

(2) Cultivation in a 50 L seed tank: inoculating the Bacillus subtilis obtained in step (1) into a seed tank filled with 35L of LB culture medium according to the inoculation amount of 8% of the volume ratio for culturing, controlling the rotation speed of the seed tank to be 400 rpm, controlling the aeration ratio to be 0.3 V/V.min, and culturing at the temperature of 30 °C for 24 h to obtain seed liquid;

(3) Production in a 5000 L fermentation tank: inoculating seed liquid into a fermentation tank filled with 3500 L of fermentation medium according to the inoculation amount of 8% volume ratio for culturing, wherein the fermentation medium comprises 40 g of bean cake powder, 50 g of bran, 60 g of corn flour, 10 g of dipotassium phosphate, 10 g of magnesium sulfate and 5 g of manganese sulfate according to the proportion for preparing to obtain the fermentation medium, adjusting the pH value to 7.8 by using sodium hydroxide solution, and fixing the volume to 1000 ml by using deionized water; controlling the rotation speed of the fermentation tank to be 800 rpm, controlling the aeration ratio to be 1.8 V/V.min, controlling the pH to be 8 at the temperature of 30 °C, culturing for 72 h, and obtaining fermentation liquid after the fermentation is finished;

(4) Carrying out ultrafiltration on the fermentation liquid obtained in step (3) by adopting a ceramic membrane with a pore diameter of 0.22 nm to obtain Bacillus subtilis thalli and obtain enzyme liquid;

(5) An isomeric system: 5 ml of the enzyme solution was mixed with 114 g of fructose and 109ml of deionized water, followed by reaction at 53 °C for 3.5 h. **The** psicose conversion was determined and calculated according to the method of example 2. **The** result shows that the conversion rate of the psicose produced by the technical scheme is 31.4%.

Comparative example 1

A method for producing psicose by fermentation and isomerization of Bacillus subtilis comprises the following specific implementation steps:

(1) Seed preparation: inoculating the Bacillus subtilis implanted with the Closridium gene prepared in example 1 into an LB medium, culturing for 24 h at the LB medium temperature of 37 °C and rotation speed of 250 rpm;

(2) Cultivation in a 50 L seed tank: inoculating the Bacillus subtilis obtained in step (1) into a seed tank filled with 35 L of LB culture medium according to the inoculation amount of 8% of the volume ratio for culturing, controlling the rotation speed of the seed tank to be 200 rpm, controlling the aeration ratio to be 0.3 V/V.min, and culturing at the temperature of 30 °C for 24 h to obtain seed liquid;

(3) Production in a 5000 L fermentation tank: inoculating seed liquid into a fermentation tank filled with 3500 L of fermentation medium for culture according to the inoculation amount of 8% volume ratio, wherein the preparation method of the fermentation medium comprises the steps of mixing 40 g of bean cake powder, 50 g of bran, 60 g of corn flour, 10 g of dipotassium phosphate, 10 g of magnesium sulfate and 5 g of manganese sulfate, adjusting the pH to 7.8 by using a sodium hydroxide solution, and fixing the volume to 1000 ml by using deionized water; controlling the rotation speed of the fermentation tank to be 100 rpm, controlling the aeration ratio to be 1.8 V/V.min, controlling the pH to be 8 at the temperature of 30 °C, culturing for 72 h, and obtaining fermentation liquid after the fermentation is finished;

(4) Separating the Bacillus subtilis thalli from the fermentation liquid obtained in step (3) by adopting a ceramic membrane ultrafiltration method with a pore diameter of 0.22 nm;

(5) An isomeric system: 5 ml of Bacillus subtilis cells as an enzyme solution were mixed with 114 g of fructose and 109 ml of deionized water, followed by reaction at 53 °C for 3.5 h. The psicose conversion was determined and calculated according to the method of example 2. The result shows that the conversion rate of the psicose produced by the technical scheme is 18.4%.

Comparative example 2

A method for producing psicose by fermentation and isomerization of Bacillus subtilis comprises the following specific implementation steps:

(1) Seed preparation: inoculating the Bacillus subtilis implanted with the Closridium gene prepared in example 1 into an LB medium, culturing for 24 h at the LB medium temperature of 37 °C and rotation speed of 250 rpm;

(2) Cultivation in a 50 L seed tank: inoculating the Bacillus subtilis obtained in step (1) into a seed tank filled with 35 L of LB culture medium according to the inoculation amount of 8% of the volume ratio for culturing, controlling the rotation speed of the seed tank to be 400 rpm, controlling the aeration ratio to be 0.45 V/V.min, and culturing at the temperature of 30 °C for 24 h to obtain seed liquid;

(3) Production in a 5000 L fermentation tank: inoculating seed liquid into a fermentation tank filled with 3500 L of fermentation medium for culture according to the inoculation amount of 8% volume ratio, wherein the preparation method of the fermentation medium comprises the steps of mixing 40 g of bean cake powder, 50 g of bran, 60 g of corn flour, 10 g of dipotassium phosphate, 10 g of magnesium sulfate and 5 g of manganese sulfate, adjusting the pH to 7.8 by using a sodium hydroxide solution, and fixing the volume to 1000 ml by using deionized water; controlling the rotation speed of the fermentation tank to be 800 rpm, controlling the aeration ratio to be 3.4 V/V.min, controlling the pH to be 8 at the temperature of 30 °C, culturing for 72 h, and obtaining fermentation liquid after the fermentation is finished;

(4) Carrying out ultrafiltration on the fermentation liquid obtained in step (3) by adopting a ceramic membrane with a pore diameter of 0.22nm to obtain Bacillus subtilis thalli and obtain enzyme liquid;

(5) An isomeric system: 5ml of the enzyme solution was mixed with 114g of fructose and 109ml of deionized water, followed by reaction at 53 °C for 3.5 h. **The** psicose conversion was determined and calculated according to the method of example 2. **The** result shows that the conversion rate of the psicose produced by the technical scheme is 21.4%.

Example 5

Enzyme activity assay

According to the conversion rate of the psicose in the examples 2-4 and the comparative examples 1-2, the enzyme activity of D-psicose-3-epimerase under different fermentation processes is calculated, and specifically calculated according to a formula I.

$$X = \frac{A \times 100 \times 9.5 \times 10^{-3} \times 10^6}{180.16 \times 10 \times 0.5} \qquad \text{Formula I}$$

In formula I:

X is the enzyme activity of D-psicose-3-epimerase, and the unit is U/mL;
A --- peak area ratio of D-psicose in liquid chromatogram, unit is %;
100 g/L --- fructose concentration, unit is g/L;
9.5 mL --- fructose volume added to the reaction system, unit is milliliter (mL);
$10^{-3}$ --- unit conversion factor, converting mL to L;
$10^6$ --- unit conversion factor, converting mol to $\mu$mol;
180.16cmolar mass of D-psicose, g/mol;
10 --- reaction time, unit is minute (min);
0.5 --- the amount of enzyme solution to be tested, unit is milliliter (mL).

The results are shown in Table 5.

Table 5. Enzyme Activity results for D-psicose-3-epimerase from different fermentation Processes

| Treatment | Example 2 | Example 3 | Example 4 | Comparative Example 1 | Comparative Example 2 |
|---|---|---|---|---|---|
| Enzyme activity (U/mL) | 309.115 | 314.39 | 331.27 | 194.12 | 225.77 |

As can be seen from the results of the above examples and comparative examples, through the exploration of fermentation process, this invention enables the high enzyme activity of D-psicose-3-epimerase to be obtained from recombinant Bacillus subtilis by a single feeding during fermentation, and the yield of psicose is ensured in fructose isomerization catalytic reaction. Compared with the experimental result of a comparative example, the ventilation volume, fermentation temperature, fermentation time, rotation speed and the culture medium are strictly controlled in the seed liquid culture and fermentation culture stages, so that higher enzyme activity is ensured, and the yield of the psicose is further improved.

**The** above description of the embodiments is only intended to facilitate the understanding of the method of the invention and its core idea. It should be noted that, for those skilled in the art, without departing from the principle of the present invention, it is possible to make various improvements and modifications to the present invention, and those improvements and modifications also fall within the scope of the claims of the present invention. Various modifications to these embodiments will be readily apparent to those skilled in the art, and the generic principles defined herein may be applied to other embodiments without departing from the spirit or scope of the invention. Thus, the present invention is not intended to be limited to the embodiments shown herein but is to be accorded the widest scope consistent with the principles and novel features disclosed herein.

**Claims**

1. A method for producing psicose by fermentation and isomerization of Bacillus subtilis **characterized by** comprising the following steps:

   Inoculating recombinant Bacillus subtilis 1A751-DPE seed liquid for expressing D-psicose 3-epimerase into a fermentation culture medium for fermentation culture to obtain a fermentation liquid; during the fermentation culture period, the aeration ratio is 0.1-2 V/V.min; the rotation speed of the fermentation culture is 200-1000 rpm;
   Separating thalli from the fermentation liquid;
   Carrying out catalytic reaction on a substrate fructose by the thalli to obtain psicose.

2. The method according to claim 1, wherein the fermentation medium comprises the following components: 10-40 g/L of bean cake powder, 20-50 g/L of bran, 30-60 g/L of corn flour, 1-10 g/L of dipotassium hydrogen phosphate, 1-10 g/L of magnesium sulfate and 0.1-5 g/L of manganese sulfate, wherein the pH value of the fermentation medium is 6-8.

3. The method according to claim 2, wherein the fermentation medium comprises the following components: 20-35 g/L of bean cake powder, 35-45 g/L of bran, 45-55 g/L of corn flour, 4-8 g/L of dipotassium hydrogen phosphate, 5-8 g/L of magnesium sulfate and 1-3 g/L of manganese sulfate, wherein the pH value of the fermentation medium is 6.5-7.5.

4. The method according to claim 1, wherein the temperature of the fermentation culture is 30-37 °C, and the time of the fermentation culture is 48-72 h;

   During fermentation, controlling the pH value of the fermentation system to be 6-8;
   The inoculation amount of the recombinant Bacillus subtilis 1A751-DPE seed liquid for expressing the D-psicose 3-epimerase is 1-10%.

5. The method according to claim 1, wherein the system of the catalytic reaction is as follows: 1-5 ml of the thalli suspension, 50-150 g of fructose and 50-200 ml of water; The thalli concentration of the thalli suspension is $(30-48) \times 10^9$ CFU/ml.

6. The method according to claim 1, wherein the temperature of the catalytic reaction is 50-60 °C;
   The time of the catalytic reaction is 2-4 h.

7. The method according to claim 1, wherein the culture medium of the recombinant Bacillus subtilis 1A751-DPE seed liquid for expressing D-psicose 3-epimerase is LB medium; The culture temperature of the recombinant Bacillus subtilis 1A751-DPE seed liquid for expressing the D-psicose 3-epimerase is 30-37 °C;

   The culture time of the recombinant Bacillus subtilis 1A751-DPE seed liquid for expressing the D-psicose 3-epimerase is 20-24 h;
   The culture rotation speed of the recombinant Bacillus subtilis 1A751-DPE seed liquid for expressing the D-psicose 3-epimerase is 200-400 rpm;
   When the recombinant Bacillus subtilis 1A751-DPE seed liquid for expressing the D-psicose 3-epimerase is cultured, the aeration ratio is 0.1-1 V/V.min;
   The inoculation amount of the recombinant Bacillus subtilis 1A751-DPE seed liquid for expressing the D-psicose 3-epimerase is 1-10%.

8. The method according to claim 1, wherein the method for separating the thalli is ceramic membrane ultrafiltration;
   The pore diameter of the ceramic membrane of the ceramic membrane ultrafiltration is 0.22 nm or less.

FIG.1

FIG.2

FIG.3

FIG.4

FIG.5

## INTERNATIONAL SEARCH REPORT

| International application No. |
|---|
| **PCT/CN2022/133876** |

**A.  CLASSIFICATION OF SUBJECT MATTER**

C12P19/24(2006.01)i;  C12P19/02(2006.01)i;  C12N15/61(2006.01)i;  C12N15/75(2006.01)i;  C12N1/21(2006.01)i; C12R1/125(2006.01)n

According to International Patent Classification (IPC) or to both national classification and IPC

**B.  FIELDS SEARCHED**

Minimum documentation searched (classification system followed by classification symbols)

IPC: C12P C12N C12R

Documentation searched other than minimum documentation to the extent that such documents are included in the fields searched

Electronic data base consulted during the international search (name of data base and, where practicable, search terms used)

CNABS, DWPI, VEN, WPABS, CNTXT, ENTXT, ENTXTC, EPTXT, OETXT, CNKI, WEB OF SCIENCE: 枯草芽孢杆菌, D-阿洛酮糖, 阿洛酮糖, 发酵, 果糖, 异构, D-allulose, D-psicose, Bacillus subtilis, D-psicose, allulose, piscose, ferment+, fructose, isomerism, isomerase 等.

**C.  DOCUMENTS CONSIDERED TO BE RELEVANT**

| Category* | Citation of document, with indication, where appropriate, of the relevant passages | Relevant to claim No. |
|---|---|---|
| X | CN 104894047 A (JIANGNAN UNIVERSITY) 09 September 2015 (2015-09-09) see abstract, and description, embodiment 3 | 1-8 |
| A | CN 113801832 A (QINGDAO VLAND BIOTECH INC.; QINGDAO VLAND BIOTECH GROUP CO., LTD.) 17 December 2021 (2021-12-17) see claims 1-7 | 1-8 |
| A | CN 105368767 A (JIANGNAN UNIVERSITY) 02 March 2016 (2016-03-02) see claims 1-7 | 1-8 |
| A | EP 3480318 A1 (ROQUETTE FRERES) 08 May 2019 (2019-05-08) see claims 1-15 | 1-8 |
| A | WO 2022179591 A1 (TIANJIN INSTITUTE OF INDUSTRIAL BIOTECHNOLOGY, CHINESE ACADEMY OF SCIENCES) 01 September 2022 (2022-09-01) see claims 1-14 | 1-8 |

☐ Further documents are listed in the continuation of Box C.  ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | "&" document member of the same patent family |
| "P" document published prior to the international filing date but later than the priority date claimed | |

| Date of the actual completion of the international search | Date of mailing of the international search report |
|---|---|
| **10 August 2023** | **23 August 2023** |

| Name and mailing address of the ISA/CN | Authorized officer |
|---|---|
| **China National Intellectual Property Administration (ISA/ CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

International application No.

**PCT/CN2022/133876**

| Box No. I | Nucleotide and/or amino acid sequence(s) (Continuation of item 1.c of the first sheet) |

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT
### Information on patent family members

International application No.

**PCT/CN2022/133876**

| Patent document cited in search report | | | Publication date (day/month/year) | Patent family member(s) | | | Publication date (day/month/year) |
|---|---|---|---|---|---|---|---|
| CN | 104894047 | A | 09 September 2015 | None | | | |
| CN | 113801832 | A | 17 December 2021 | None | | | |
| CN | 105368767 | A | 02 March 2016 | None | | | |
| EP | 3480318 | A1 | 08 May 2019 | MX | 2020004692 | A | 20 August 2020 |
| | | | | WO | 2019086708 | A1 | 09 May 2019 |
| | | | | EP | 3707270 | A1 | 16 September 2020 |
| | | | | US | 2023151347 | A1 | 18 May 2023 |
| | | | | KR | 20200078526 | A | 01 July 2020 |
| | | | | JP | 2021501595 | A | 21 January 2021 |
| | | | | JP | 7165730 | B2 | 04 November 2022 |
| WO | 2022179591 | A1 | 01 September 2022 | None | | | |

Form PCT/ISA/210 (patent family annex) (July 2022)